Europäisches Patentamt

European Patent Office

Office européen des brevets

(11) Numéro de publication: **0 305 229 B1**

# FASCICULE DE BREVET EUROPEEN

(45) Date de publication de fascicule du brevet: **22.06.94**

(51) Int. Cl.5: **C12N 15/00**, C12N 7/00, C12N 5/00, C12P 21/02, A61K 39/165, A61K 39/285, A61K 39/395

(21) Numéro de dépôt: **88401750.0**

(22) Date de dépôt: **05.07.88**

(54) **Vecteur viral et ADN recombinant codant pour une ou des protéines de structure (HA, F et/ou NP), d'un morbillivirus.**

(30) Priorité: **07.07.87 FR 8709629**

(43) Date de publication de la demande:
**01.03.89 Bulletin 89/09**

(45) Mention de la délivrance du brevet:
**22.06.94 Bulletin 94/25**

(84) Etats contractants désignés:
**AT BE CH DE ES FR GB GR IT LI LU NL SE**

(56) Documents cités:
**EP-A- 0 276 591**

**PROCEEDINGS OF THE NATIONAL ACADEMY OF SCIENCES, vol. 83, no. 19, octobre 1986, pages 7462-7466, Washington, DC, US; R.A. OLMSTED et al.: "Expression of the Fglycoprotein of respiratory syncytial virus by a recombinant vaccinia virus: Comparison of the individual contributions of the F and G glycoproteins to host immunity"**

(73) Titulaire: **TRANSGENE S.A.**
**11, rue de Molsheim**
**F-67000 Strasbourg(FR)**

(72) Inventeur: **Drillien, Robert**
**10, Bd Paul Déroulède**
**F-67000 Strasbourg(FR)**
Inventeur: **Spehner, Danièle**
**29, rue de la Chênaie**
**F-67200 Eckbolsheim(FR)**
Inventeur: **Wild, Fabian**
**27, rue Guilloud**
**F-69003 Lyon(FR)**
Inventeur: **Lecocq, Jean-Pierre**
**6, rue du Champ du Feu**
**F-67116 Reichstett(FR)**

(74) Mandataire: **Warcoin, Jacques et al**
**Cabinet Régimbeau**
**26, avenue Kléber**
**F-75116 Paris (FR)**

J. GEN. VIROL., vol. 68, no. 6, juin 1987, pages 1695-1703, GB; R. BUCKLAND et al.: "Fusion glycoprotein of measles virus: nucleotide sequence of the gene and comparison with other paramyxoviruses"

J. GEN. VIROL., vol. 67, no. 12, décembre 1986, pages 2695-2703, GB; C. GERALD et al.: "Measles virus haemagglutinin gene: cloning, complete nucleotide sequence analysis and expression in COS cells"

PROCEEDINGS OF THE NATIONAL ACADEMY OF SCIENCES, vol. 85, février 1988, pages 1252-1256; R. DRILLIEN et al.: "Protection of mice from fatal measles encephalitis by vaccination with vaccinia virus recombinants encoding either the hemagglutinin or the fusion protein"

BIOCHEMICAL AND CELLULAR BIOLOGY, vol. 64, no. 10, 1986, pages 1038-1043; P.A.GREER et al.: "Cloning and in vitro expression of the measles virus matrixgene"

## Description

Le virus de la rougeole est responsable d'une maladie humaine très contagieuse caractérisée par des symptômes divers (conjonctivite, toux et éruption cutanée). Le système nerveux central peut également être atteint en donnant lieu soit à une encéphalite soit de manière plus rare une panencéphalite subaiguë sclérosante. Les différentes formes de la maladie peuvent être évitées grâce à la vaccination avec un virus de la rougeole atténué selon des méthodes traditionnelles. Bien que les vaccins actuellement employés donnent pour une large part satisfaction car ils permettent d'abaisser de manière spectaculaire le nombre des cas de rougeole ainsi que le nombre de décès ou complications dûs à ce virus, plusieurs problèmes subsistent qui justifient l'amélioration de la prévention anti-rougeoleuse. Une première difficulté est liée au fait que les vaccins utilisés actuellement sont constitués de virus vivant atténué, dont l'administration pose plus de problèmes que celle d'un vaccin à base d'organismes tués. Les efforts réalisés jusqu'à présent pour mettre au point un vaccin tué ont tous échoué. Par ailleurs, les vaccins actuellement utilisés sont relativement sensibles à la chaleur en raison des propriétés physico-chimiques du virus ce qui rend leur emploi difficile dans les pays tropicaux. Or la rougeole est l'une des premières causes de décès chez l'enfant dans de nombreux pays à climat tropical.

Les travaux antérieurs (Norrby, 1985) sur le virus de la rougeole et les analogies que l'on peut établir avec d'autres virus appartenant à la même famille donnent à penser que les protéines de surface de la particule virale, à savoir la protéine hémagglutinante (HA) et la protéine de fusion (F), sont suffisantes pour induire une immunité protectrice.

Par ailleurs, les relations immunologiques entre les protéines de surface du virus de la rougeole et celles des autres Morbillivirus (5, 1, 4, 8, 9) et les homologies de séquences entre les ADN codant pour certaines de ces protéines (10) permettent d'envisager l'utilisation d'antigènes du virus de la rougeole pour la vaccination contre la maladie de Carré, la peste bovine et la peste des petits ruminants. Il est également possible de prévoir l'utilisation des antigènes de surface provenant de chacun des Morbillivirus en système de vaccination homologue ou hétérologue.

D'autre part, plusieurs travaux ont montré que les protéines internes de différents virus sont importantes pour l'induction d'une immunité de type cellulaire. Pour compléter le vaccin contre les Morbillivirus, il peut donc être important d'introduire, dans le virus recombinant, le gène codant pour la nucléoprotéine (NP).

C'est pourquoi la présente invention concerne un Poxvirus recombinant caractérisé en ce qu'il comporte, dans une ou plusieurs parties non essentielles de son génome, au moins une séquence d'ADN codant pour tout ou partie d'au moins une protéine de structure d'un Morbillivirus ainsi que les éléments assurant l'expression de cette protéine.

Les Morbillivirus qui peuvent être utilisés sont choisis parmi le virus de la rougeole, le virus de la maladie de Carré, le virus de la peste bovine et le virus de la peste des petits ruminants.

Parmi les Poxvirus utilisables, il faut citer plus particulièrement le virus de la vaccine.

Les protéines de structure plus particulièrement intéressantes sont les protéines de surface, la protéine hémagglutinante (HA), la protéine de fusion (F) et une protéine interne, la nucléoprotéine (NP). Les séquences d'ADN correspondantes peuvent coder pour la protéine de longueur totale ou bien pour des fragments suffisants pour induire des anticorps neutralisant le virus de la rougeole ou les autres Morbillivirus ou une réponse immunitaire de type cellulaire.

Il est évidemment possible de prévoir la mise en oeuvre d'une séquence d'ADN codant pour plusieurs protéines ou plusieurs fragments de celles-ci.

Bien que la souche du virus de la rougeole mise en oeuvre dans les exemples du brevet soit une souche particulière, il est possible d'utiliser d'autres souches. En effet, étant donné la conservation importante des épitopes antigéniques associés aux protéines HA et F des différentes souches du virus de la rougeole, la méthode décrite pour une souche de virus peut être étendue aux autres souches.

Les éléments d'expression de la protéine sont essentiellement des promoteurs, en particulier des promoteurs efficaces dans la vaccine, ainsi on peut utiliser le promoteur du gène codant pour la protéine de 7,5 Kd de la vaccine qui sera appelé en abrégé promoteur 7,5 Kd.

L'insertion de l'ADN doit se faire dans une partie non essentielle du génome du virus, c'est-à-dire dans une partie qui n'empêche pas la réplication du Poxvirus.

On effectue par exemple l'intégration dans le gène TK de la vaccine car, outre le fait qu'il n'est pas essentiel pour le virus, ce gène permet une sélection des virus ayant intégré la séquence en cause.

On peut aussi effectuer l'intégration dans un gène dénommé hr qui n'est nécessaire à la multiplication du virus que dans certains types cellulaires.

Suivant les cas, les différentes protéines de Morbillivirus peuvent être exprimées simutanément par le même virus recombinant ou par plusieurs virus. Dans le premier cas, les gènes correspondant peuvent

être intégrés au même site ou à des sites différents, en particulier dans les gènes TK et hr.

C'est pourquoi la présente invention concerne également des virus recombinants caractérisés en ce qu'ils comportent simultanément dans 2 parties non essentielles de leur génome des séquences d'ADN codant pour différentes protéines de Morbillivirus.

Les Poxvirus recombinants ainsi obtenus, qu'ils soient vivants ou inactivés, sont plus particulièrement utilisables comme vaccins, notamment contre la rougeole, la maladie de Carré, la peste bovine et la peste des petits ruminants.

Toutefois, il est possible d'utiliser ces Poxvirus de façon indirecte pour infecter des cellules de mammifères, in vitro, afin d'obtenir des protéines de structure de Morbillivirus, notamment de la rougeole, qui pourraient être utilisées comme agents d'immunisation et de vaccination ou encore à titre d'éléments de diagnostic selon des procédés connus.

C'est pourquoi l'invention concerne également l'application des protéines de structure ou de fragments de protéines de structure à titre d'agents vaccinants et qui peuvent notamment être obtenues à partir des cultures cellulaire précédentes.

Enfin, l'invention concerne également les antisérums contenant les anticorps dressés contre les poxvirus ou les protéines selon l'invention, pour leur utilisation en sérothérapie.

Les figures suivantes illustrent les exemples :

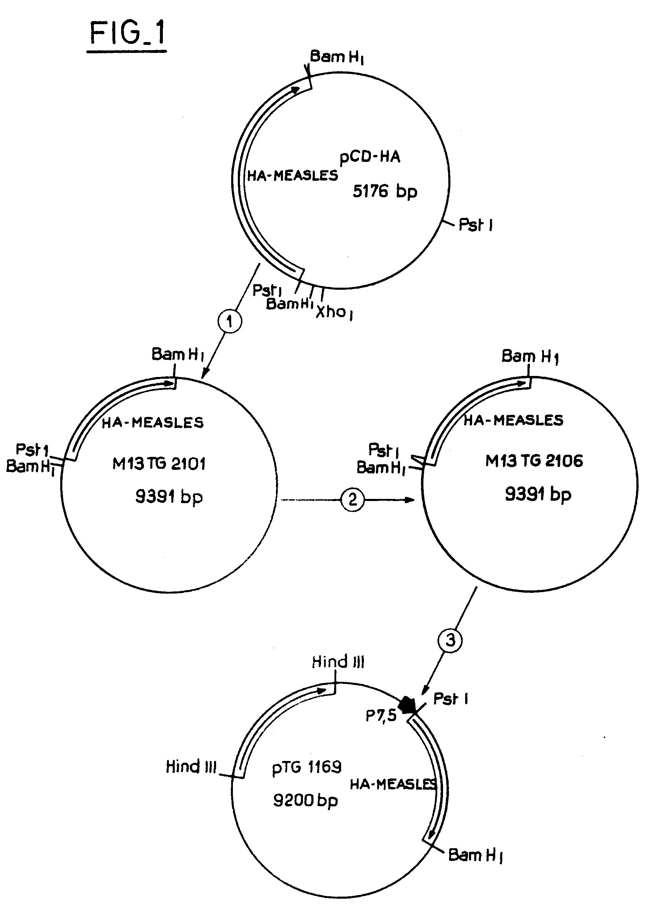

Figure 1 : Construction du vecteur permettant le transfert du cADN codant pour la protéine HA dans le génome du virus de la vaccine.

La séquence rougeole (HA measles) est indiquée par un arc ouvert avec une flèche qui donne le sens de la traduction du gène. Le grand fragment BamHI dans pCD-HA est le vecteur pCD tandis que dans M13TG2101 et M13TG2106 le grand fragment BamHI est le vecteur M13TG131. Dans l'étape 3 le plasmide recombinant M13TG2107 n'est pas représenté. Dans le plasmide pTG1169 le petit fragment HindIII correspond à pTG1H, le trait continu au fragment HindIIIJ du virus vaccinal interrompu dans le gène TK et la flèche large correspond au promoteur de la protéine 7,5 Kd.

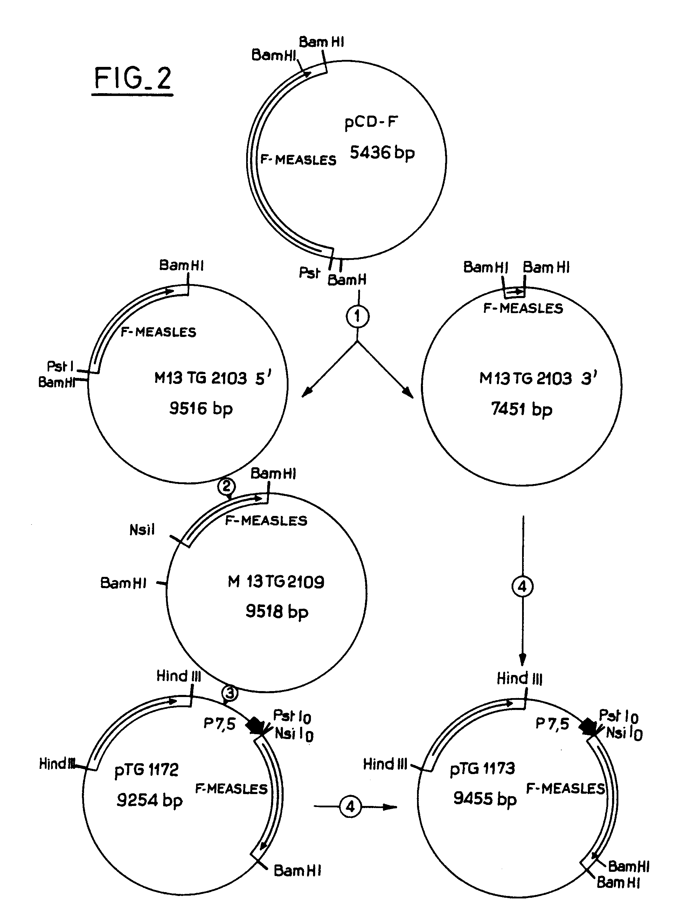

Figure 2 : Construction du vecteur permettant le transfert du cADN codant pour la protéine F dans le génome du virus de la vaccine.

La séquence rougeole (F measles) est indiquée par un arc ouvert avec une flèche qui donne le sens de la traduction. Les autres annotations sont identiques à celles employées dans la figure 1.

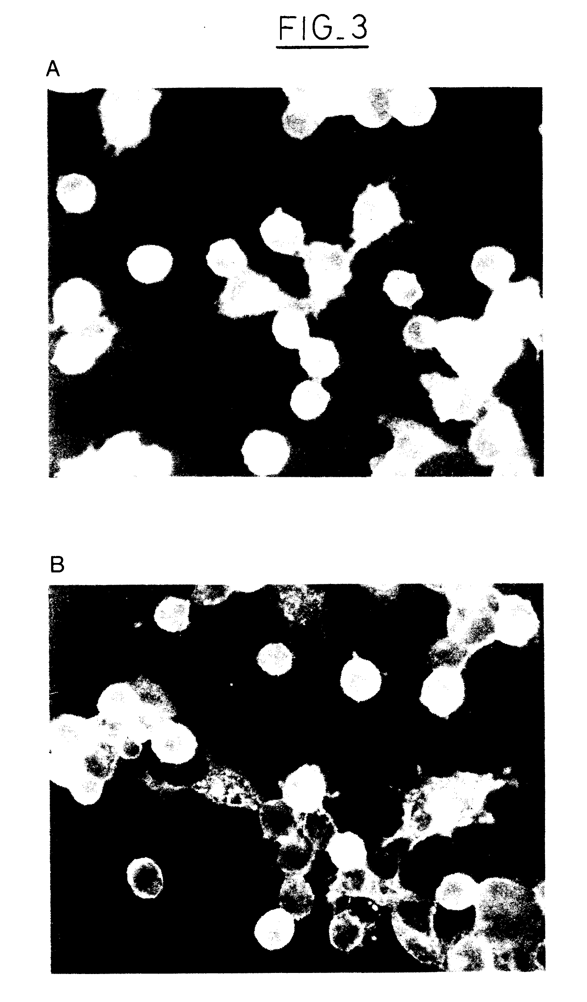

Figure 3 : Protéines HA et F du virus de la rougeole synthétisées dans des cellules infectées par les recombinants du virus de la vaccine et mises en évidence par immunofluorescence.

Le panneau A montre l'immunofluorescence membranaire obtenue après infection de cellules LTK⁻ avec le recombinant VV.TG.HAM2-2 et réaction d'un anticorps monoclonal anti-HA suivi d'un anticorps anti-souris marqué à la fluorescéine. Le panneau B montre l'immunofluorescence observée après infection des cellules LTK⁻ avec le recombinant VV.TG.FM1173 et réaction d'un anticorps monoclonal anti F suivi d'un anticorps anti-souris marqué à la fluorescéine.

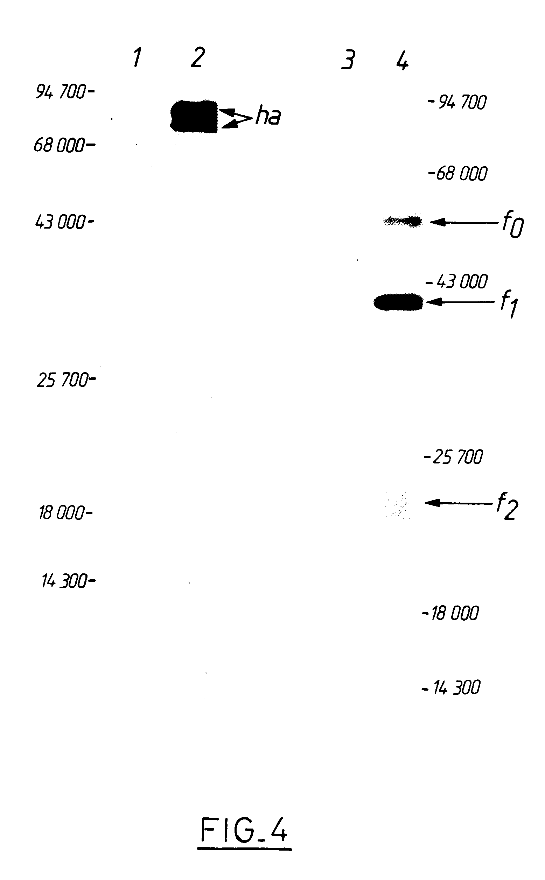

Figure 4 : Immunoprécipitation des protéines HA et F du virus de la rougeole synthétisées dans des cellules infectées par des recombinants du virus de la vaccine.

Autoradiographie du gel de polyacrylamide sur lequel les protéines immunoprécipitées ont été déposées. L'hémaglutinine (HA) est présente dans les cellules infectées avec VV.TG.HAM2-2 tandis que la protéine de fusion (F0) et ses produits de clivage F1 et F2 sont présents dans les cellules infectées avec VV.TG.FM1173.

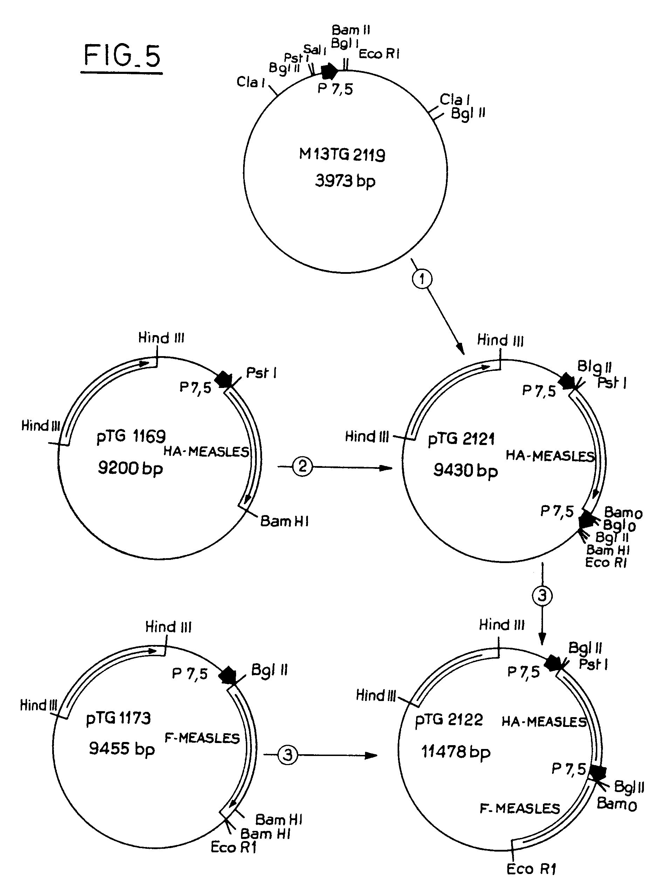

Figure 5 : Construction du vecteur permettant le transfert du cADN codant pour la protéine F et HA dans le génome du virus de la vaccine.

Les annotations sont identiques à celles employées dans les figures 1 et 2.

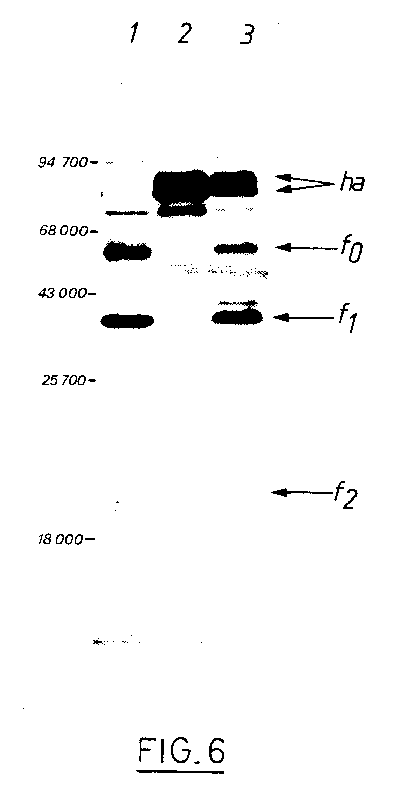

Figure 6 : Immunoprécipitation des protéines HA et F du virus de la rougeole synthétisées dans des cellules infectées par le recombinant vv.TG.HAFM2122 codant pour les deux protéines.

Autoradiographie du gel de polyacrylamide sur lequel les protéines immunoprécipitées ont été déposées. Les extraits de cellules infectées avec VV.TG.FM1173 (colonne 1), VV.TG.HAM2-2 (colonne 2) et VV.TG.HAFM2122 (colonne 3) ont été immunoprécipitées avec un sérum polyclonal de cobaye. Les protéines HA, F0, F1 et F2 attendues après infection avec le recombinant VV.TG.HAFM2122 apparaissent distinctement dans la colonne 3.

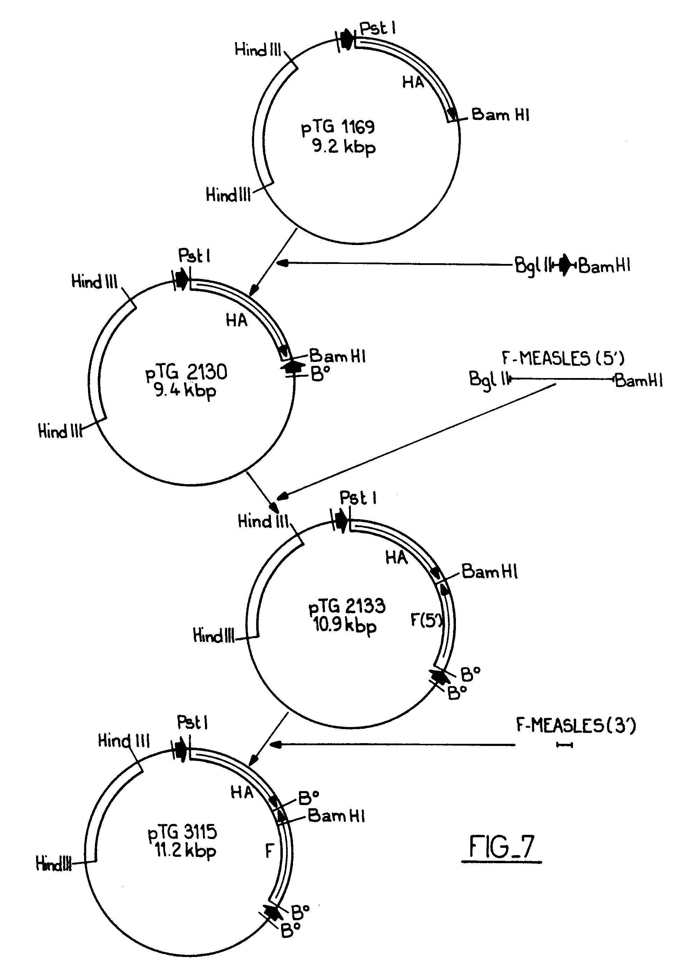

Figure 7 : Construction d'un deuxième vecteur permettant le transfert du cADN codant pour la protéine F et HA dans le génome du virus de la vaccine.

Les séquences du virus de la rougeole codant pour les protéines HA et F sont représentées par des arcs de cercles annotés HA et F. Les gènes sont orientés selon le sens donné par les flèches. Le promoteur P7,5 Kd du virus de la vaccine est schématisé par une flèche épaisse. La région d'ADN comprise entre les sites HindIII correspond au vecteur bactérien pTG1H. En amont et en aval du vecteur bactérien sont représentés par un arc de cercle simple, la partie gauche et la partie droite respectivement du fragment HindIII J du virus de la vaccine. Le symbole B° indique l'endroit où un site BglII a été fusionné par ligation à un site BamHI.

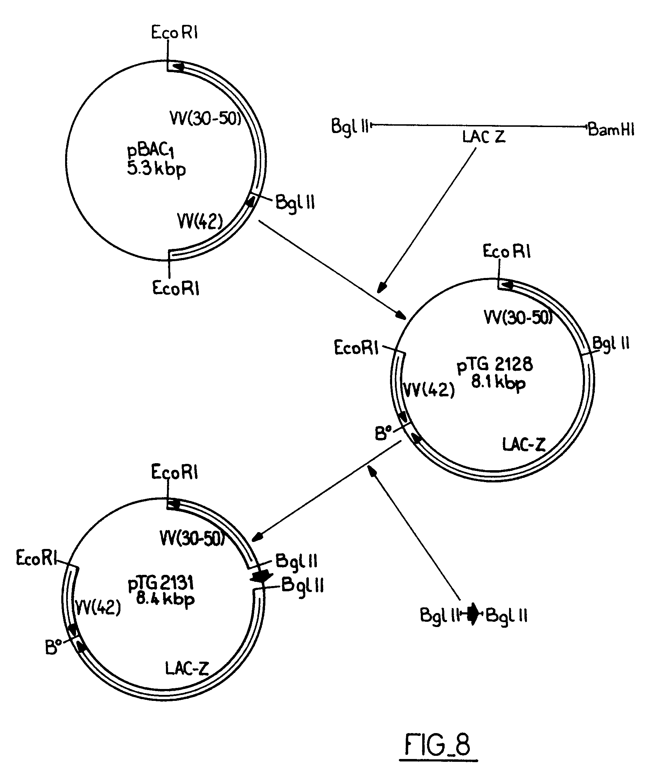

Figure 8 : Construction d'un vecteur permettant le transfert du gène codant pour la $\beta$-galactosidase d'E. coli à la place du gène hr dans le génome du virus de la vaccine.

La partie correspondant au vecteur bactérien pUC$_7$ est représentée par un arc de cercle au trait unique. Les séquences d'ADN du virus de la vaccine situées de part et d'autre du site BglII sont représentées par des arcs de cercle ou trait double et correspondent aux régions du génome qui codent pour des protéines de 30 Kd et 50 Kd (à gauche du site BglII) ou 42 Kd (à droite du site BglII). Le gène codant pour la $\beta$-galactosidase est annoté lac Z et son orientation est donnée par rapport au promoteur P7,5 Kd. Ce dernier est représenté par une flèche épaisse. Le symbole B° indique l'endroit où un site BglII a été fusionné par ligation à un site BamHI.

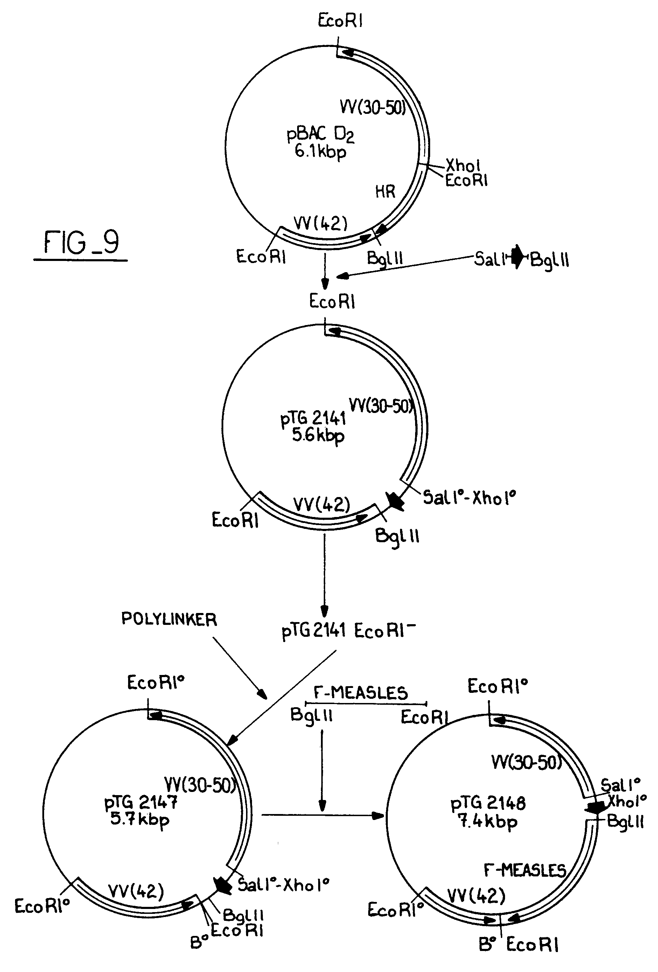

Figure 9 : Construction d'un vecteur permettant le transfert du gène codant pour la protéine F du virus de la rougeole à la place du gène hr dans le génome du virus de la vaccine.

Les annotations sont les mêmes que celles utilisées dans la figure 8. Le symbole E° indique l'endroit du vecteur où le site EcoR1 a été supprimé. La région située entre le site BglII et le site EcoR1 du vecteur pTG2147 correspond au segment adaptateur. Le gène de la rougeole codant pour la protéine F est représenté par un arc de cercle en traits doubles et annoté Measles F.

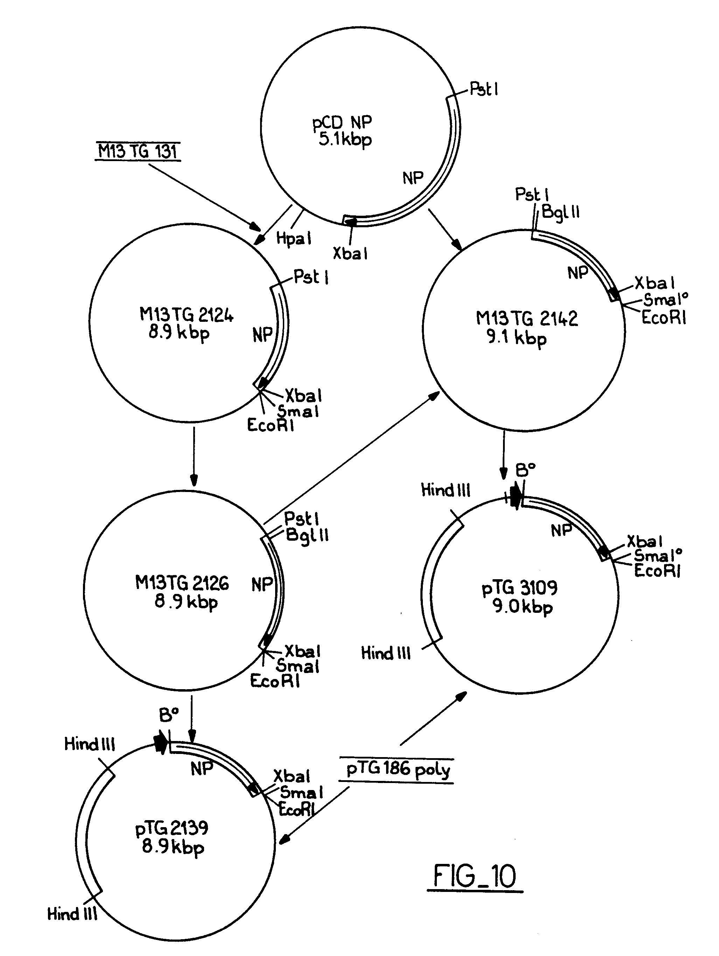

Figure 10 : Construction de deux vecteurs permettant le transfert du gène codant pour la protéine NP du virus de la rougeole dans le génome du virus de la vaccine.

Dans le plasmide pCD NP, les arcs de cercle au trait simple représentent la partie pCD. Dans les plasmides annotés M13, les régions au trait unique représentent la partie M13TG131. Les plasmides pTG sont schématisés comme dans les figures précédentes. Les sites ayant été détruits par ligation sont annotés B° pour la fusion d'un site BglII à un site BamHI et Sma 1° pour la fusion d'un site Sma1 à un site Hpa1.

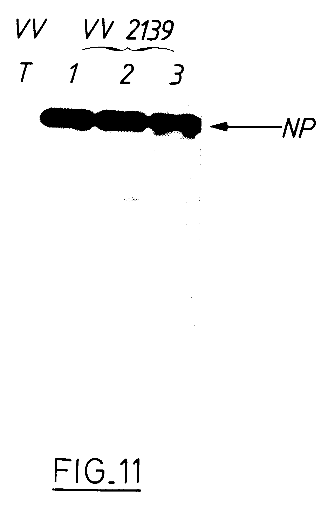

Figure 11 : Mise en évidence de la protéine NP dans des cellules infectées par le virus VV.TG.2139.

Les lysats de cellules de hamster BHK 21 infectées avec la souche sauvage du virus de la vaccine (piste annotée T) ou avec des isolats du recombinant VV.TG.2139 (annotés 1,2 et 3) ont été immunoprécipités avec un sérum polyclonal de cobaye et analysés par électrophorèse sur gel de polyacrylamide. L'autoradiographie du gel révèle la présence d'une protéine NP (60 Kd) seulement dans les échantillons de cellules infectées avec le virus VV.TG.2139.

Exemple 1 Construction d'un vecteur permettant le transfert du cADN codant pour la protéine HA dans le génome du virus de la vaccine.

Le cADN codant pour la protéine HA du virus de la souche Hallé a été isolé et séquencé précédemment (3). Afin de faciliter la manipulation du gène, le cADN HA provenant du plasmide pCD HA a été excisé par les deux sites BamHI qui l'entourent et inséré dans le vecteur M13TG131 (6) pour donner naissance au plasmide M13TG2101. Un site Pst1 a été introduit par mutagénèse localisée en amont de la partie codante du gène HA portée par le plasmide M13TG2101 à l'aide de l'oligonucléotide synthétique suivant :

GGGGGGGGGAGGGCTGCAGATCATCCACAATG

Le plasmide muté a été dénommé M13TG2106. Un fragment Pst1-BamHI contenant le gène HA a été excisé du M13TG2106 et intégré dans le vecteur M13TG130 (6) préalablement coupé par Pst1-BamHI pour donner naissance au plasmide M13TG2107. La séquence nucléotidique de la région mutée du cADN HA a été vérifiée par la méthode des didéoxynucléotides puis le cADN HA a été excisé du vecteur M13TG2107 avec les enzymes Pst1-EcoRI et inséré en aval du promoteur 7,5 Kd du vecteur de transfert pTG186POLY (brevet français 84.06499) préalablement coupé par les enzymes Pst1-EcoRI pour donner naissance au plasmide pTG1169. L'ensemble de ces étapes est schématisé dans la figure 1.

Exemple 2 Construction d'un vecteur permettant le transfert du cADN codant pour la protéine F dans le génome du virus de la vaccine.

Le cADN codant pour la protéine F du virus de la souche Hallé a été isolé et séquencé précédemment (2). Afin de faciliter la manipulation du gène, le cADN codant pour la protéine F a été excisé du vecteur pCD F (2) à l'aide de l'enzyme BamHI. Ceci donne deux fragments, l'un qui recouvre la partie 5′ du gène et l'autre qui recouvre la partie 3′ du gène. Le fragment 5′ a été inséré dans le vecteur M13TG131 préalablement coupé par l'enzyme BamHI pour donner le plasmide M13TG2103-5. La partie 3′ a été de même intégrée dans le vecteur M13TG131 pour donner M13TG2103-3′. Un site Nsil a été introduit par mutagénèse localisée en amont de la partie codante du gène F porté par le plasmide M13TG2103-5′ à l'aide de l'oligonucléotide synthétique suivant :

## Nsil

## AAGACTCATCCAATGCATATCATGGGTCTCAAG

Le plasmide muté a été dénommé M13TG2109. Un fragment Nsi1-BamHI contenant la partie 5′ du gène F a été excisé du M13TG2109 et inséré en aval du promoteur 7,5 Kd du vecteur de transfert pTG186POLY préalablement coupé par les enzymes Pst1-BamHI pour donner naissance au vecteur pTG1172. La partie 3′ du gène F a été excisée de M13TG2103-3′ par l'enzyme BamHI et insérée dans l'orientation qui reconstitue le gène naturel dans pTG1172 préalablement coupé par BamHI pour donner le vecteur pTG1173. L'ensemble de ces étapes est schématisé dans la figure 2.

Exemple 3 Construction d'un recombinant du virus de la vaccine contenant le gène HA du virus de la rougeole et capable d'exprimer la protéine correspondante.

Le gène codant pour la protéine HA contenu sur le plasmide pTG1169 a été transféré dans le génome du virus de la vaccine selon un protocole classique précédemment décrit (brevet français 84.06499). En bref, des cellules d'embryon de poulet ont été infectées avec le mutant thermosensible du virus de la vaccine ts N7 à raison de 0,1 pfu par cellule et incubées pendant deux heures à 33°C (température permissive pour le mutant). Les cellules ont été ensuite transfectées avec un précipité de phosphate de calcium contenant 1 μg d'ADN de la souche sauvage du virus de la vaccine et 100 ng du plasmide pTG1169. Après une heure d'incubation à température ambiante, un milieu de culture frais a été ajouté aux cellules sans enlever le précipité et l'incubation a été poursuivie pendant deux heures à la température de 39,5°C (température non permissive pour le mutant). Au bout de cette période le milieu contenant le précipité a été éliminé et remplacé pendant une minute par un milieu de culture contenant 10 % de glycerol. Enfin les cellules ont été lavées deux fois avec du PBS puis incubées pendant deux jours à 39,5° dans un milieu de culture normal. Des virus recombinants, thymidine kinase négatif ont ensuite été sélectionnés, à partir du virus non thermosensible produit dans la transfection précédente. Pour réaliser cette étape les cellules de poulet infectées et transfectées de la manière décrite ci-dessus ont été lysées par congélation puis sonication et le virus qu'elles contiennent titré sur cellules LTK⁻ en présence de 100

6

μg/ml de bromodésoxyuridine et sous une couche d'agarose 1 %. Des plages de virus TK⁻ ont été reprises et le virus qu'elles contiennent a été amplifié sur des cultures fraîches de cellules d'embryon de poulet. Les stocks de virus ainsi obtenus ont été analysés pour leur aptitude à synthétiser de la protéine HA, soit par une technique d'immunofluorescence, soit par une technique d'immunoprécipitation.

Pour l'immunofluorescence des cellules LTK⁻ ont été infectées avec le virus recombinant à raison d'environ une pfu par cellule pendant 15 heures. Les cellules infectées ont ensuite été fixées à l'acétone, réhydratées au PBS puis incubées pendant une demi-heure avec un anticorps monoclonal de souris dirigé contre la protéine HA du virus de la rougeole et dilué au 1/200e. Après lavage au PBS pour éliminer l'anticorps non fixé, les cellules ont été incubées en présence d'anticorps anti-souris marqué à la fluorescéine et dilué au 1/100e. Après de nouveaux lavages pour éliminer l'anticorps marqué et fixé aspécifiquement, les échantillons sont observés au microscope à fluorescence. La photo de la figure 3 montre que les cellules infectées avec le recombinant isolé ci-dessus présentent une fluorescence caractéristique de cellules infectées par le virus de la rougeole tandis que les cellules infectées par du virus de la vaccine sauvage ne présentent pas cette fluorescence. Un des recombinants du virus de la vaccine capable de synthétiser la protéine HA a été choisi et dénommé VV.TG.HAM2-2. Pour s'assurer qu'il ne contenait pas de contamination par un autre virus non recombinant il a été purifié une nouvelle fois par isolement d'une plage infectieuse et amplification sur cellules d'embryon de poulet.

Afin de confirmer la synthèse de la protéine HA d'une taille attendue, des cellules BHK21 ont été infectées à raison d'environ 0,1 pfu par cellule pendant 15 heures puis incubées dans un milieu dépourvu de méthionine normale mais contenant de la méthionine radioactive marquée au soufre 35. Après quatre heures de marquage, les cellules infectées ont été lysées dans du tampon d'immunoprécipitation et la protéine HA immunoprécipitée avec un anticorps polyclonal de cobaye dirigé contre les protéines du virus de la rougeole, en présence de la protéine A de Staphylocoque aureus fixée au sépharose. Les protéines immunoprécipitées ont été analysées sur un gel de polyacrylamide en présence de SDS. Le gel séché est soumis à autoradiographie. Les résultats (figure 4) montrent que le recombinant du virus de la vaccine VV.TG.HAM2-2 induit la synthèse d'une protéine de taille identique à la protéine HA authentique du virus de la rougeole.

Exemple 4 Construction d'un recombinant du virus de la vaccine contenant le gène codant pour la protéine F du virus de la rougeole.

Le gène codant pour la protéine F porté par le plasmide pTG1173 a été transféré dans le génome du virus de la vaccine selon le même protocole que celui décrit dans l'exemple 3 pour le gène HA. Ainsi un recombinant viral dénommé VV.TG.FM1173 a été isolé. Afin de confirmer que ce recombinant induit la synthèse de la protéine F, des cellules BHK21 ont été infectées avec environ 0,1 ufp par cellule pendant 15 heures puis incubées dans un milieu dépourvu de méthionine normale mais contenant de la méthionine radioactive marquée au $^{35}$S. Après quatre heures de marquage, les cellules infectées ont été lysées dans du tampon d'immunoprécipitation et la protéine F immunoprécipitée avec un anticorps polyclonal de cobaye dirigé contre les protéines du virus de la rougeole, en présence de la protéine A de Staphylocoque aureus fixée au sépharose.

Les protéines immunoprécipitées ont été analysées sur gel de polyacrylamide en présence de SDS. Le gel séché est soumis à autoradiographie. Les résultats (figure 4) montrent que le recombinant VV.TG.FM1173 induit la synthèse de la protéine F0 correspondant au produit initial de la traduction du gène F et des produits de clivage protéolytique F1 et F2 qui apparaissent naturellement lors d'une infection par la rougeole. Les protéines F0, F1 et F2 ont la taille attendue pour les protéines authentiques du virus de la rougeole. Afin de montrer que ces protéines sont associées à la membrane de la cellule infectée comme lors de l'infection par le virus de la rougeole, des cellules LTK⁻ ont été infectées avec le recombinant VV.TG.FM1173 à raison d'environ une ufp par cellule pendant 15 heures. Les cellules infectées ont ensuite été fixées à l'acétone, réhydratées au PBS puis incubées pendant une demi-heure avec un anticorps monoclonal de souris dirigé contre les protéines F du virus de la rougeole et dilué au 1/100e. Après lavage au PBS les cellules ont été incubées en présence d'anticorps anti-souris marqués à la fluorescéine et dilué au 1/100e. Après de nouveaux lavages pour éliminer l'anticorps marqué et fixé aspécifiquement, les échantillons sont observés au microscope à fluorescence. La photo de la figure 3 montre que les cellules infectées avec le recombinant VV.TG.FM1173 présentent une fluorescence membranaire caractéristique de cellules infectées par le virus de la rougeole tandis que les cellules infectées avec le virus de la vaccine de type sauvage ne présentent aucune fluorescence.

<u>Exemple 5</u> Vaccination des souris avec le recombinant VV.TG.HAM2-2.

Des souris Balb C âgées de 4 à 5 semaines ont été vaccinées par scarification de la queue avec 4 $10^7$ pfu de virus, soit du recombinant VV.TG.HAM2-2, soit du virus VV sauvage. Trois semaines après l'inoculation du sang a été prélevé des animaux et on a mesuré le taux d'anticorps capables d'inhiber l'activité hémagglutinante du virus et capables de neutraliser le virus in vitro. Le tableau 1 montre que la plupart des souris vaccinées avec VV.TG.HAM2-2 présentent aussi bien des anticorps anti-hémagglutinants que des anticorps neutralisants. Trois semaines après la vaccination une inoculation d'épreuve a été administrée aux animaux : chaque souris a reçu 50 $\mu$l d'une suspension diluée à 10 % d'un cerveau de souris préalablement infectée avec une souche de rougeole neuroadaptée (11). Les animaux ont été surveillés pendant un mois. Le tableau 2 montre que les souris vaccinées avec la souche VV.TG.HAM2-2 sont protégées tandis que les autres animaux succombent à l'inoculation d'épreuve.

<u>Exemple 6</u> Vaccination des souris avec le recombinant VV.TG.FM1173.

Des souris Balb C femelles âgées de cinq semaines ont été vaccinées par scarification de la queue avec 4.$10^7$ ufp de virus, soit du recombinant VV.TG.FM1173, soit du virus VV sauvage. Vingt cinq jours après l'inoculation, du sang a été prélevé des animaux et on a mesuré le taux d'anticorps capables de neutraliser le virus in vitro. Le tableau 3 montre que la plupart des souris vaccinées avec VV.TG.FM1173 présentent des anticorps neutralisants. Le jour du prélèvement sanguin les souris ont été inoculées par voie intracérébrale avec une souche virulente neuroadaptée du virus de la rougeole. Chaque souris reçoit 50 $\mu$l d'une suspension diluée à 10 % d'un cerveau de souris préalablement infectée avec la souche virulente neuroadaptée. Les animaux sont surveillés pendant un mois. Seules les souris vaccinées avec la souche VV.TG.FM1173 sont protégées contre l'inoculation d'épreuve (Tableau 2).

Tableau 1

| Immunisation de souris par inoculation du virus recombinant VV.TG.HAM2-2. | | |
|---|---|---|
| <u>souris femelles</u> | anticorps anti-hémagglutinine | anticorps neutralisants |
| 1 | 320 | 1280 |
| 2 | 80 | 160 |
| 3 | 160 | 80 |
| 5 | 320 | 1280 |
| 6 | 40 | 80 |
| 7 | 20 | 40 |
| 8 | 40 | 40 |
| 9 | 160 | 160 |
| 10 | 40 | - |
| <u>souris mâles</u> | | |
| 1 | 160 | 80 |
| 2 | 160 | 80 |
| 3 | 160 | 320 |
| 4 | 160 | 320 |
| 5 | 80 | 640 |
| 6 | 160 | 320 |
| 7 | 160 | 320 |
| 8 | 160 | 160 |
| 9 | 40 | 80 |
| 10 | 80 | 80 |
| 11 | 80 | 80 |
| 12 | 80 | 320 |
| 13 | 80 | 320 |
| 14 | 80 | 320 |
| 15 | 80 | 80 |

Les titres sont exprimés en réciproque de la dilution la plus élevée du sérum qui donnait une neutralisation complète de 50 ufp du virus de la rougeole ou une inhibition totale de l'activité hémagglutinante. Les sérums de souris non vaccinées ou vaccinées avec la souche VV sauvage donnaient un titre d'anticorps anti-hémagglutinine inférieur à 20 et un titre d'anticorps neutralisants inférieur à 40.

**Tableau 2**    **Protection des souris.**

| virus utilisé pour la vaccina-tion | Expérience 1 | | Expérience 2 | | |
|---|---|---|---|---|---|
| | VV sauvage | VV.TG.HAM2-2 | pas de virus | VV sauvage | VV.TG.FM1173 |
| nombre d'animaux inoculés | 12 | 19 | 9 | 3 | 22 |
| nombre* de survi-vants | 2 | 19 | 1 | 0 | 22 |

* Trois semaines après la vaccination, les souris ont été éprouvées avec une souche SSPE de la rougeole adaptée à la souris. Les décès ayant eu lieu dans les trois premiers jours n'ont pas été comptabilisés dans les résultats car ils sont attribués au traumatisme de la voie d'inoculation.

Tableau 3

| Immunisation des souris par inoculation du virus recombinant VV.TG.FM1173. | |
| --- | --- |
| Souris femelles | Anticorps neutralisants |
| 1 | 160 |
| 2 | 160 |
| 3 | 160 |
| 4 | 320 |
| 5 | 320 |
| 6 | 160 |
| 7 | 160 |
| 8 | 80 |
| 9 | 20 |
| 10 | 40 |
| 11 | 20 |
| 12 | 20 |
| 13 | 80 |
| 14 | 160 |
| 15 | 40 |
| 16 | 160 |
| 17 | 160 |
| 18 | 80 |
| 19 | 320 |
| 20 | 160 |
| 21 | 160 |
| 22 | 40 |
| 23 | 40 |
| 24 | 40 |

Les titres sont exprimés en réciproque de la dilution la plus élevée du sérum qui donnait une neutralisation complète de 50 ufp du virus de la rougeole. Les sérums de souris non vaccinées ou vaccinées avec la souche sauvage donnaient un titre d'anticorps neutralisants inférieur à 40.

Exemple 7 Construction d'un recombinant du virus de la vaccine contenant les gènes codant pour la protéine F et pour la protéine HA du virus de la rougeole.

Un plasmide recombinant permettant le transfert simultané des gènes HA et F dans le génome du virus de la vaccine a été construit de la manière suivante (illustré dans la figure 5) : le promoteur 7,5 Kd a été excisé du vecteur M13TG2119 par digestion partielle avec l'enzyme BglII et digestion complète avec EcoRI. Le fragment BglII-EcoRI ainsi obtenu a été inséré dans le plasmide pTG1169 préalablement coupé par BamHI et EcoRI. De cette manière un nouveau plasmide a été isolé et appelé pTG2121.

Dans cette construction, le gène HA est suivi en 3′ par le promoteur 7,5 Kd orienté dans le même sens de transcription que le gène HA.

Le plasmide pTG2121 a été ouvert en aval du second promoteur 7,5 Kd avec les enzymes BamHI et EcoRI pour y insérer le gène F, excisé du plasmide pTG1173 par les enzymes BglII et EcoRI ; le nouveau plasmide est appelé pTG2122. Ainsi le plasmide pTG2122 correspond au plasmide pTG186POLY dans lequel ont été insérés les gènes HA et F, chacun étant placé sous le contrôle d'un promoteur 7,5 Kd. L'ensemble a été transféré dans le génome du virus de la vaccine selon le protocole décrit dans l'exemple 3. Un virus recombinant (VV.TG.HAFM2122) capable d'induire la synthèse de la protéine HA et de la protéine F a été isolé et caractérisé par immunofluorescence et immunoprécipitation comme décrit dans les exemples 3 et 4 (voir figure 6 pour l'immunoprécipitation).

Exemple 8 Construction d'un deuxième recombinant du virus de la vaccine contenant les gènes codant pour la protéine F et pour la protéine HA du virus de la rougeole.

Le recombinant VV.TG.HAFM2122 s'est révélé instable. En effet, l'analyse de la population virale issue d'un clonage du recombinant a montré que tandis que 100 % d'entre eux codent toujours pour la protéine F, seulement 50 % codent pour la protéine HA. Un clonage successif du même type en partant toujours d'un clone viral codant pour les deux protéines a donné des résultats identiques. Il est probable que la présence du promoteur 7,5 Kd en répétition directe permette la délétion du gène (le gène HA en l'occurence) qui est encadré par la répétition.

Afin de générer un recombinant codant pour les protéines HA et F de manière stable, les deux promoteurs 7,5 Kd ont été placés en répétition inversée de telle sorte que toute recombinaison donnerait naissance à un génome viral tronqué, non viable. Ceci constitue donc un système de sélection contre la délétion du gène HA et assure la stabilité du virus recombinant.

Les constructions ont été réalisées de la manière suivante. Dans un premier temps un deuxième promoteur 7,5 Kd a été ajouté au plasmide pTG1169. Le promoteur a été excisé de M13TG2119 en coupant celui-ci de manière partielle par BglII et de manière complète par BamHI. Le fragment d'ADN contenant le promoteur coupé par BglII en amont et par BamHI en aval a été isolé et lié au plasmide pTG1169 préalablement coupé par BamHI et traité à la phosphatase.

Après transformation de bactéries E. coli avec le mélange de ligation, un plasmide pTG2130 a été isolé dans lequel le deuxième promoteur 7,5 Kd est dans le sens de transcription opposé au premier. L'extrémité codant pour la partie N terminale de la protéine F a ensuite été excisée de pTG1173 par les enzymes BglII et BamHI et insérée dans le vecteur pTG2130 préalablement coupé par BamHI et déphosphorylé. Ceci donne naissance au plasmide pTG2133.

Afin d'ajouter la partie C terminale du gène F il s'est révélé nécessaire de déléter auparavant l'extrémité poly A qui se trouvait dans le cADN d'origine et avait été transférée au vecteur M13TG2103 3′. Pour ce faire, une mutagénèse localisée avec l'oligonucléotide

5′.AATTATCTCCGGCTTAGATCTGGCCGAACAATATCGG

a été effectuée de façon à introduire un site BglII à 246 nucléotides en aval du site BamHI situé dans le gène F. Le phage M13 muté appelé M13TG2143 a servi de source d'un fragment BamHI-BglII codant pour la séquence C terminale du gène F. Celui-ci a été inséré dans le plasmide pTG2133 coupé au préalable par BamHI puis déphosphorylé. Le plasmide recombinant obtenu nommé pTG3115 comprend le gène HA et F placés en sens opposés l'un par rapport à l'autre, chacun étant controlé par un promoteur p7,5 Kd également opposés l'un par rapport à l'autre. L'ensemble des manipulations effectuées pour construire le plasmide pTG3115 est résumé dans la figure 7.

Le transfert des séquences décrites ci-dessus dans le génome du virus de la vaccine a été effectué selon les méthodes décrites auparavant et un virus appelé VV.TG.HAFM3115 a été isolé. Ce virus recombinant code pour les protéines HA et F d'après les critères d'immunofluorescence et d'immunoprécipi-tation utilisés ci-dessus et par ailleurs il présente un phé-notype (expression des protéines HA et F) stable après clonages successifs.

Exemple 9 Construction d'un recombinant du virus de la vaccine codant pour la protéine F du virus de la rougeole en remplacement du gène hr

Des travaux antérieurs (12) ont montré que le virus de la vaccine possède un gène, dénommé gène hr, qui est nécessaire à la multiplication virale sur certains types cellulaires d'origine humaine mais n'est pas indispensable sur d'autres types cellulaires (cellules d'embryons de poulet, cellules BHK21 de hamster, cellules L de souris). Ces résultats indiquent que le gène hr pourrait être délété et remplacé par un autre gène.

Le gène qui code pour la protéine F du virus de la rougeole a été intégré à la place du gène hr de la manière suivante :

1° Intégration du gène de la β galactosidase à la place du gène hr dans le génome du virus de la vaccine.

Dans un premier temps un plasmide a été construit dans lequel le gène hr est remplacé par un bloc d'expression comprenant le promoteur p7,5 Kd suivi du gène de la β-galactosidase. L'ADN viral qui se trouve en amont et en aval du gène hr (ce dernier ayant été délété) a été introduit dans le vecteur pUC7 pour donner le pBAC1. Le site BglII unique de ce plasmide correspond au site où était localisé le gène hr.

Le plasmide pBAC1 a été ouvert par Bg1II et un fragment Bg1II-BamH1 provenant de pTG1174 et contenant le gène de la $\beta$ galactosidase y a été inséré de telle sorte que le sens de la lecture du gène soit de la gauche vers la droite par rapport au sens conventionnel du génome viral. Le plasmide obtenu, pTG2128 a été ouvert au site Bg1II unique en amont du gène de la $\beta$-galactosidase et le promoteur p7,5 Kd contenu sur un fragment d'ADN Bg1II-Bg1II provenant de M13TG2119 a été intégré dans le sens permettant la transcription du gène de la $\beta$ galactosidase. Le plasmide provenant de cette ligation est appelé pTG2131 ; les étapes de sa construction sont schématisées dans la figure 8.

Des cellules d'embryon de poulet infectées au préalable par le mutant tsN7 du virus de la vaccine ont été transfectées simultanément par l'ADN de virus de type sauvage et par l'ADN du pTG2131. Les virus non thermosensibles issus de cette expérience ont été étalés sur cellules d'embryons de poulet pour la formation de plages sous une couche de milieu contenant de l'agar à 1 %. Deux jours après, on ajoute une deuxième couche de milieu contenant de l'agar et 60 mg par ml de X-Gal (5-bromo-4chloro-3indoly-$\beta$-D-galactopyranoside).Cette technique permet de reconnaître les plages bleues contenant du virus qui a intégré et qui exprime le gène de la $\beta$-galactosidase.

Parmi les nombreux virus recombinants reconnus de cette manière, une plage a été isolée et le virus a été recloné puis amplifié et dénommé VV.TG.hr$\beta$gal.2131. L'étude de son spectre d'hôte sur plusieurs types cellulaires montre qu'il ne se multiplie pas sur cellules RK13 de lapin ni sur cellules humaines 143B tk$^-$ . Ce phénotype correspond à ce que l'on attend pour un virus ayant perdu le gène hr. Par contre le virus VV.TG.hr$\beta$gal.2131 est toujours capable de se multiplier sur cellules humaines HepII.

2° Construction d'un virus de phénotype hr$\beta$gal thermosensible

Afin de disposer d'un virus qui possède à la fois un phénotype ts et une substitution du gène hr par le gène de la $\beta$-galactosidase on a réalisé une infection mixte de cellules d'embryon de poulet avec le mutant VV tsN7 et le virus VV.TG.hr$\beta$gal.2131. Après infection avec 5 ufp par cellule de chaque virus les cellules ont été incubées à 33°C pendant un jour. Les cellules ont ensuite été congelées puis décongelées et le lysat titré sur cellules d'embryon de poulet à 33°C. Après deux jours à 33°C les cellules infectées ont été colorées au rouge neutre puis transférées à 39,5°C pendant deux jours supplémentaires. Les plages de virus qui ne se sont pas agrandies pendant les deux derniers jours sont vraisemblablement des virus de type ts. Une quarantaine de ceux-ci ont été repris, amplifiés puis criblés à la fois pour leur caractère ts et pour la capacité de former des plages bleues en présence de X-gal. Cinq recombinants ts$\beta$galhr ont été identifiés de cette manière et l'un d'entre eux appelé VV.TG.ts$\beta$galhr.1 a été recloné et utilisé dans les expériences suivantes.

3° Construction d'un plasmide vecteur destiné à l'intégration d'un gène quelconque dans le génome du virus de la vaccine en remplacement du gène hr

Le gène hr du virus de la vaccine entouré de part et d'autre par ses séquences adjacentes naturelles a été cloné dans le plasmide bactérien pUC7. Cette construction a été appelée pBACD2. Dans ce plasmide, le gène hr a été modifié de façon à présenter à ses extrémités un site Xho1 et un site Bg1II.

Le gène hr a été délété en coupant pBACD2 avec XhoI et Bg1II puis le promoteur 7,5 Kd entouré par des sites SalI et Bg1II (provenant de M13TG2119) a été inséré à sa place. Le plasmide obtenu est appelé pTG2141. Dans ce plasmide, le promoteur 7,5 Kd est orienté de la gauche vers la droite, selon la convention adoptée pour le génome du virus de la vaccine. Le mode d'intégration choisi a pour effet de supprimer le site Xho1 en amont du promoteur et de conserver le site Bg1II en aval.

Le plasmide pTG2141 a ensuite été modifié afin de supprimer les sites EcoR1 situés aux extrémités de la partie pUC7. Ainsi l'ADN plasmidique de pTG2141 a été digéré par EcoR1 en concentration limitante de façon à ne couper le plasmide qu'une fois. Le vecteur linéarisé a été isolé puis traité par le fragment Klenow de l'ADN-polymérase en présence des quatre nucléosides triphosphates puis fermé à la ligase. Après transformation de cellules d'E. coli un plasmide ne contenant plus qu'un site EcoR1 a été isolé et celui-ci a subi le même traitement à l'enzyme EcoR1 pour donner un nouveau plasmide sans site EcoR1, appelé PTG2141-EcoR1.

Ce dernier a été coupé par Bg1II pour y insérer un segment d'ADN adaptateur provenant du vecteur Poly II (13) qui est entouré par les sites Bg1II et BamH1 ; cette construction est appelée pTG2147. Le vecteur pTG2147 comprend donc une région promoteur (p7,5 Kd) suivie d'une série de sites de restriction qui peuvent être utilisés pour ajouter un gène quelconque. Les différentes étapes de la construction de ce vecteur sont schématisées dans la figure 9.

4° Intégration du gène F du virus de la rougeole en remplacement du gène hr dans le génome du virus de la vaccine

Le plasmide pTG2147 a été ouvert avec les enzymes Bg1II et EcoR1 pour y intégrer un fragment d'ADN, Bg1II-EcoR1 provenant de pTG1173, codant pour la protéine F du virus de la rougeole. Le plasmide obtenu, pTG2148, a été employé pour substituer le gène de la $\beta$-galactosidase dans le virus

VV.TG.hr$\beta$gal.2131 par le gène F du virus de la rougeole.

Pour cela, des cellules d'embryons de poulet ont été infectées avec le virus VV.TG.ts$\beta$galhr.1 puis transfectées avec le plasmide pTG2148 et de l'ADN purifié du virus VV.TG.hr$\beta$gal.2131 Après incubation des cellules pendant 48 heures à 39,5 °C, le virus non thermosensible issu de l'expérience a été étalé sur cellules d'embryons de poulet sous un milieu contenant de l'agar à 1 %. Deux jours plus tard du milieu contenant de l'agar et 60 mg par ml de X-gal a été ajouté et les virus des plages qui n'ont pas donné de couleur bleue ont été repris et amplifiés.

Ces derniers proviennent vraisemblablement de la recombinaison du virus non thermosensible portant le gène de la $\beta$-galactosidase avec le plasmide qui contient le gène F du virus de la rougeole. Un virus recombinant issu de cette expérience et appelé VV.TG.FM2148 induit en effet la synthèse de la protéine F dans les cellules infectées, selon les critères d'immunofluorescence employés précédemment.

Exemple 10 Construction d'un recombinant du virus de la vaccine contenant les gènes codant pour les protéines HA et F du virus de la rougeole dans deux régions distinctes du génome, tk et hr.

On a sélectionné de nouveaux virus recombinants portant le gène HA à la place du gène vaccinal Tk et le gène F à la place du gène vaccinal hr.

Des cellules d'embryons de poulet ont été coinfectées avec le virus VV.TG.FM2148 et avec le virus VV.TG.HAM2-2. Après 24 heures d'infection les cellules ont été congelées, décongelées puis le lysat étalé à différentes dilutions sur des cellules Ltk⁻ en présence de 5′ bromodésoxyuridine. Cette première étape permet de sélectionner les virus recombinants tk⁻ ayant par conséquent conservé le gène HA intégré dans le gène tk.

Des cellules d'embryons de poulet ont été infectées avec les virus issus des plages isolées lors de cette étape. Après deux jours d'incubation les cellules ont été colorées avec du rouge neutre et les plages présentant un aspect caractéristique du phénotype hr ont été reprises et le virus amplifié par infection de cellules d'embryons de poulet.

L'un des nombreux virus recombinants isolés a été retenu et appelé VV.TG.HA/tk.F/hr. Ce dernier induit la synthèse de la protéine F et de la protéine HA, selon le critère d'immunofluorescence employé auparavant.

Exemple 11 Construction de deux recombinants du virus de la vaccine contenant le gène NP du virus de la rougeole et capables d'exprimer la protéine correspondante.

Une banque de cADN faite à partir de RNA de cellules Vero infectées avec le virus de la rougeole (souche Hallé) a été construite dans le vecteur pCD (2,3). Un cADN codant pour la nucléoprotéine (NP) a été sélectionné par hybridation à l'aide d'un cADN homologue de celui décrit par Gorecki et Rosenblatt (14). Ce cADN codant pour la nucléoprotéine (NP), cloné dans le vecteur pCD1, donne le plasmide pCDNP.

La détermination de la séquence nucléotidique du gène NP de la souche Hallé met en évidence sa parenté étroite avec la séquence du gène NP d'un autre isolat du virus de la rougeole publiée par Rozenblatt et al. (15). Toutefois, on remarque un G supplémentaire à la position 1489 dans la séquence de Rozenblatt, également détecté par Cattaneo et al. (16). Cette modification change le cadre de lecture publié pour les 29 acides aminés à l'extrémité carboxy terminale.

Par ailleurs, la séquence à l'extrémité 5′ du gène NP qui n'a pas été publiée auparavant a été déterminée afin de faciliter la mutagénèse localisée décrite plus loin. Le cADN codant pour la protéine NP (environ 1620 nucléotides) a été excisé de pCDNP par les enzymes Pst1 et Xba1 puis inséré dans le vecteur M13TG131 ouvert par les mêmes enzymes pour donner le phage M13TG2124. Le cADN codant pour NP et porté par M13TG2124 a été modifié par mutagénèse dirigée à l'aide de l'oligonucléotide :

CCTTAAAAGTGTGGCCATCTTAGATCTCCCTAATCCTGCTC

pour introduire un site Bg1II trois nucléotides en amont du premier codon du gène NP. Le gène NP ainsi modifié a été excisé du phage muté, appelé M13TG2126, à l'aide du site Bg1II nouvellement créé et d'un site EcoR1 situé en 3′ du gène NP.

Le fragment Bg1II-EcoR1 codant pour NP a ensuite été inséré en aval du promoteur 7,5 Kd porté par le vecteur pTG186 poly (13) préalablement coupé par BamH1 et EcoR1 ce qui donne le plasmide pTG2139. L'utilisation, du site Xba1 pour les clonages décrits ci-dessus entraine une délétion d'un codon du gène NP et crée un gène de fusion dans lequel les 22 derniers codons proviennent en partie du vecteur M13 utilisé et en partie du gène tk du virus de la vaccine.

Afin de réaliser une construction analogue à pTG2139 mais contenant un gène NP complet tout en étant dépourvu de codons d'une autre origine, la partie carboxy terminale du gène NP a été excisée de pCDNP par les enzymes Xba1 et Hpa1 et le fragment obtenu a été inséré dans M13TG2126 coupé par Xba1 et Sma1 pour donner M13TG2142. Puis le cADN complet codant pour NP a été sorti de M13TG2142 par Bg1II-EcoR1 et intégré dans pTG186poly coupé par BamH1 et EcoR1. Le plasmide issu de ce dernier clonage est appelé pTG3109. Les différentes étapes de la construction des vecteurs pTG2139 et pTG3109 sont schématisées dans la figure 10.

Le gène NP tronqué (plasmide pTG2139) ou complet (plasmide pTG3109) a été transféré dans le génome du virus de la vaccine selon la technique résumée dans l'exemple 3. Deux recombinants du virus de la vaccine issus de ces manipulations ont été nommés respectivement VV.TG.NP2139 et VV.TG.NP3109.

Pour vérifier qu'ils codent effectivement pour la protéine NP du virus de la rougeole, des cellules L de souris ont été infectées puis analysées 16 heures après par immunofluorescence avec un anticorps monoclonal anti NP. Cette réaction a permis de mettre en évidence la présence de l'antigène NP aussi bien dans le cytoplasme que dans le noyau des cellules infectées avec les deux virus recombinants tandis que l'infection par la souche sauvage du virus de la vaccine ne donne lieu à aucune fluorescence spécifique.

La synthèse de la protéine NP a également été mise en évidence par la technique d'immunoprécipitation suivie de l'analyse par électrophorèse sur gel de polyacrylamide. Cette méthode a permis de montrer qu'une protéine d'environ 60 Kd, ce qui correspond à la taille attendue pour la protéine NP, est synthétisée dans les cellules infectées par le recombinant VV.TG.2139 (voir figure 11).

La scarification de souris BalbC avec $4.10^7$ ufp du recombinant VV.TG.2139 a induit la synthèse d'anticorps capables d'immunoprécipiter la protéine NP de cellules infectées par le virus de la rougeole.

Dépôt de souches représentatives de l'invention

Les souches suivantes ont été déposées à la Collection Nationale de cultures de Microorganismes (25 Rue du Dr. Roux, Paris), le 3 avril 1987 :
5K/pTG1173 sous le n° I-654
5K/pTG1169 sous le n° I-657.
et le 17 juin 1988 :
5K/pTG3109 sous le N° I-769.

REFERENCES

1) Appel, M.J.G., Shek, W.R., Shesberadoran, H. & Norrby, E. Archives of Virology 82, 73-82 (1984).
2) Buckland, R., Gerald, C., Barker, R. & Wild, T.F. J. Gen. Virol. 68, 1695-1703 (1987).
3) Gerald, C., Bukland, R., Barker, R., Freemon, G. & Wild, T.F. J. Gen. Virol. 67 (1986).
4) Hall, W.W., Lamb, R.A. & Choppin, P.W. Virology 100, 433-449 (1980).
5) Imagawa, D.T. Prog. Med. Virol. vol. 10, pp. 160-193 ed. Karger, Basel/New York (1968).
6) Kieny, M.P., Lathe, R. & Lecocq, J.P. Gene 26, 91-99 (1983).
7) Norrby, E. Measles in Virology ed. B.N. Fields et al. Raven Press, New York, 55, 1305-1321 (1985)
8) Norrby, E., Sheshberodaran, H., Mc Cullough, K.C., Carpenter, W.C. & Orvell, C. Intervirology 23, 228-232 (1985).
9) Orvell, C. & Norrby, E. J. Gen. Virol. 50, 231-245 (1980).
10) Varsanyi, T., Joinvall, H., Orvell, C. and Norrby, E. Virology 157, 241-244 (1987).
11) Wild, T.F., Giraudon, P., Bernard, A. & Huppert, J. J. Med. Virol. 4, 103-114 (1979).
12) Gillard, S., Spehner, D., Drillien, R. and Kirn, Proc. Natl. Acad. Sci - USA, 83, 5573-5577 (1986)
13) Lathe, R., Vilotte, J.L., Clarck, A.J. Gene 57, 193-201 (1987)
14) Gorecki, M., Rosenblatt. Proc. Natl. Acad. Sci - USA, 77, 3686-3690 (1980)
15) Rozenblatt, S., Eizenberg, O., Ben-Levy, R., Lavie, V. and Bellini, W.J. J. Virol. 53, 684-690 (1985)
16) Cattaneo, R., Schmid, A., Billeter, M.A., Sheppard, R.D. and Udem, S.A. J. Virol. 62, 1388-1397 (1988)

**Revendications**
**Revendications pour les Etats contractants suivants : AT, BE, CH, LI, DE, FR, GB, IT, LU, NL, SE**

**1.** Poxvirus recombinant caractérisé en ce qu'il comporte, dans une ou plusieurs parties non essentielles de son génome, au moins une séquence d'ADN codant pour tout ou partie d'au moins une protéine F et/ou NP d'un Morbillivirus ainsi que ses éléments assurant l'expression de ladite protéine.

2. Poxvirus selon la revendication 1, caractérisé en ce que le Morbillivirus est choisi parmi le virus de la rougeole, le virus de la maladie de Carré, le virus de la peste bovine et le virus de la peste des petits ruminants.

3. Poxvirus selon la revendication 1 ou 2, caractérisé en ce qu'il s'agit du virus de la vaccine.

4. Poxvirus selon la revendication 3, caractérisé en ce que la séquence d'ADN est sous le contrôle d'un promoteur de la vaccine.

5. Poxvirus selon la revendication 4, caractérisé en ce que le promoteur de la vaccine est le promoteur 7,5 Kd de la vaccine.

6. Poxvirus selon la revendication 5, caractérisé en ce que la ou les séquences d'ADN sont clonées dans le gène TK de la vaccine.

7. Poxvirus selon la revendication 5, caractérisé en ce que la ou les séquences d'ADN sont clonées dans le gène hr de la vaccine.

8. Poxvirus selon la revendication 5,caractérisé en ce qu'il comporte au moins deux séquences d'ADN codant pour tout ou partie d'une proteine de structure d'un Morbillivirus, ces séquences étant intégrées dans deux régions du génome choisies parmi les gènes TK et hr.

9. ADN recombinant correspondant au poxvirus selon l'une des revendications 1 à 8.

10. Cellule de mammifère infectée par le poxvirus selon l'une des revendications 1 à 8.

11. Procédé de préparation de tout ou partie d'une protéine de structure d'un Morbillivirus, caractérisé en ce qu'on cultive des cellules selon la revendication 10 et en ce que l'on récupère ladite protéine de structure.

12. Protéine de structure de Morbillivirus susceptible d'être obtenue par le procédé selon la revendication 9.

13. A titre d'agent immunogène, les protéines selon la revendication 12, et la protéine hémagglutinante HA d'un Morbillivirus, seules ou en combinaison.

14. Antisérum contenant les anticorps dressés contre les Poxvirus selon l'une des revendications 1 à 8 ou contre les protéines selon la revendication 12.

15. Vaccin caractérisé en ce qu'il contient un Poxvirus selon l'une des revendications 1 à 8 et/ou des agents immunogènes selon la revendication 13 seules ou en combinaison et/ou un Poxvirus recombinant caractérisé en ce qu'il comporte, dans une ou plusieurs parties non essentielles de son génome, au moins une séquence d'ADN codant pour tout ou partie d'au moins une protéine hémagglutinante HA d'un Morbillivirus ainsi que ses éléments assurant l'expression de ladite protéine.

16. Vaccin selon la revendication 15, caractérisé en ce que le Poxvirus est vivant.

17. Vaccin selon la revendication 15, caractérisé en ce que le Poxvirus est inactivé.

18. Vaccin selon l'une des revendications 15 à 17, caractérise en ce que le Poxvirus est le virus de la vaccine.

19. Poxvirus selon la revendication 18, caractérisé en ce que la séquence d'ADN est sous le contrôle d'un promoteur de la vaccine.

15

**20.** Vaccin selon la revendication 19, caractérisé en ce que le promoteur de la vaccine est le promoteur 7,5 Kd de la vaccine.

**21.** Vaccin selon la revendication 20, caractérisé en ce que la ou les séquence(s) d'ADN sont clonées dans le gène TK de la vaccine.

**22.** Vaccin selon la revendication 20,caractérisé en ce que la ou les séquence(s) d'ADN sont clonées dans le gène hr de la vaccine.

**23.** Vaccin selon la revendication 20, caractérisé en ce qu'il comporte au moins deux séquences d'ADN codant pour tout ou partie d'une protéine de structure d'un Morbillivirus, ces séquences étant intégrées dans deux régions ou génome choisies parmi les gènes TK et hr.

**Revendications pour les Etats contractants suivants : ES, GR**

**1.** Procédé de préparation d'un Poxvirus recombinant caractérisé en ce qu'on introduit, dans une ou plusieurs parties non essentielles de son génome, au moins une séquence d'ADN codant pour tout ou partie d'au moimns une protéine F et/ou NP d'un Morbillivirus ainsi que ses éléments assurant l'expression de ladite protéine.

**2.** Procédé selon la revendication 1, caractérisé en ce que Morbillivirus est choisi parmi le virus de la rougeole, le virus de la maladie de Carré, le virus de la peste bovine et le virus de la peste des petits ruminants.

**3.** Procédé selon la revendication 1 ou 2, caractérisé en ce qu'il s'agit du virus de la vaccine.

**4.** Procédé selon la revendication 3, caractérisé en ce que la séquence d'ADN est sous le contrôle d'un promoteur de la vaccine.

**5.** Procédé selon la revendication 4, caractérisé en ce que la promoteur de la vaccine est le promoteur 7,5 Kd de la vaccine.

**6.** Procédé selon la revendication 5, caractérisé en ce que la ou les séquences d'ADN sont clonées dans le gène TK de la vaccine.

**7.** Procédé selon la revendication 5, caractérisé en ce que la ou les séquences d'ADN sont clonées dans le gène hr de la vaccine.

**8.** Procédé selon la revendication 5, caractérisé en ce qu'il comporte au moins deux séquences d'ADN codant pour tout ou partie d'une protéine de structure d'un Morbillivus, ces séquences étant intégrées dans deux régions du génome choisies parmi les gènes TK et hr.

**9.** Procédé de préparation de tout ou partie d'une protéine de structure d'un Morbillivirus, caractérisé en ce qu'on cultive des cellules de mammifère infectées par un poxvirus obtenu par un procédé selon l'une des revendications 1 à 8 et en ce qu'on récupère ladite protéine de structure.

**10.** Procédé de préparation d'un vaccin caractérisé en ce qu'il contient un Poxvirus obtenu selon un procédé selon l'une des revendications 1 à 8 et/ou des protéines obtenues par un procédé selon la revendication 9 seules ou en combinaison, et/ou un Poxvirus recombinant caractérisé en ce qu'il comporte, dans une ou plusieurs parties non essentielles de son génome, au moins une séquence d'ADN codant pour tout ou partie d'au moins une protéine hémagglutinante HA d'un Morbillivirus ainsi que ses éléments assurant l'expression de ladite protéine.

**11.** Procédé selon la revendication 10, caractérisé en ce que le Poxvirus est vivant.

**12.** Procédé selon la revendication 10, caractérisé en ce que le Poxvirus est inactivé.

**13.** Procédé selon l'une des revendications 10 à 12, caractérisé en ce que le Poxvirus est le virus de la vaccine.

**14.** Procédé selon la revendication 13, caractérisé en ce que la séquence d'ADN est sous le contrôle d'un promoteur de la vaccine.

**15.** Procédé selon la revendication 14, caractérisé en ce que le promoteur de la vaccine est le promoteur 7,5 Kd de la vaccine.

**16.** Procédé selon la revendication 15, caractérisé en ce que la ou les séquence(s) d'ADN sont clonées dans le gène TK de la vaccine.

**17.** procédé selon la revendication 15, caractérisé en ce que la ou les séquence(s) d'ADN sont clonées dans le gène hr de la vaccine.

**18.** Procédé selon la revendication 15, caractérisé en ce qu'il comporte au moins deux séquences d'ADN codant pour tout ou partie d'une protéine de structure d'un Morbillivirus, ces séquences étant intégrées dans deux régions ou génome choisies parmi les gènes TK et hr.

## Claims
## Claims for the following Contracting States : AT, BE, CH, LI, DE, FR, GB, IT, LU, NL, SE

**1.** Recombinant poxvirus characterized in that it comprises, in one or several unessential parts of its genome, at least one DNA sequence coding for all or part of at least one F and/or NP protein of Morbillivirus as well as the elements ensuring the expression of said protein.

**2.** Poxvirus according to claim 1, wherein the Morbillivirus is selected from among the virus of measles, the virus of Carre's disease, the virus of measles, the virus of cattle plague and the virus of small ruminants pest.

**3.** Poxvirus according to one of claims 1 or 2, comprising the virus of the vaccine.

**4.** Poxvirus according to claim 3, wherein the DNA sequence is under the control of a promoter of the vaccine.

**5.** Poxvirus according to claim 4, wherein the promoter of the vaccine is the 7.5 Kd promoter of the vaccine.

**6.** Poxvirus according to claim 5, wherein the one or more DNA sequences are cloned in the TK gene of the vaccine.

**7.** Poxvirus according to claim 5, wherein the one or more DNA sequences are cloned in the hr gene of the vaccine.

**8.** Poxvirus according to claim 5, comprising at least two DNA sequences coding for all or part of a structural protein of a Morbillivirus, said sequences being integrated in two regions of the genome selected from among the TK and hr genes.

**9.** Recombinant DNA corresponding to the poxvirus according to one of claims 1 to 8.

**10.** Mammalian cell infected with the poxvirus according to one of claims 1 to 8.

**11.** Process for the preparation of all or part of a structural protein of a Morbillivirus, said process comprising cultivating cells according to claim 10 and recovering said structural protein.

**12.** Morbillivirus structural protein obtainable by the process according to claim 9.

**13.** As an immunogenic agent, the proteins according to claim 12, and the HA hemoglutinating protein alone or in combination.

**14.** Antiserum containing antibodies set up against the poxviruses according to one of claims 1 to 8 or against proteins according to claim 12.

**15.** Vaccine which contains a poxvirus according to one of claims 1 to 8 and/or immunogenic agents according to claim 13 alone or in combination and/or a recombinant poxvirus characterized in that it comprises, in one or several unessential parts of its genome, at least one DNA sequence coding for all or part of at least a hemogglutinating protein of a morbillivirus as well as elements ensuring the expression of said protein.

**16.** Vaccine according to claim 15, wherein the poxvirus is living.

**17.** Vaccine according to claim 15, wherein the poxvirus is inactivates.

**18.** Vaccine according to claim 15 to 17, characterized in that the poxvirus is the virus of the vaccine.

**19.** Vaccine according to claim 18, characterized in that the DNA sequence is under the control of a promoter of the vaccine.

**20.** Vaccine according to claim 19, characterized in that the promoter of the vaccine is the 7.5 Kd promoter.

**21.** Vaccine according to claim 20, characterized in that the one or more DNA sequences are cloned in the TK gene of the vaccine.

**22.** Vaccine according to claim 20, characterized in that the one or more DNA sequences are cloned in the hr gene of the vaccine.

**23.** Vaccine according to claim 20, characterized in that it comprises at least two DNA sequences coding for all or part of a structural protein of a Morbillivirus, said sequences being integrated in two regions of the genome selected among the TK and hr genes.

**Claims for the following Contracting States : ES, GR**

**1.** Process for preparing a recombinant poxvirus characterized in that one introduces, in one or several unessential parts of its genome, at least one DNA sequence coding for all or part of at least one F or NP protein of Morbillivirus as well as the elements ensuring the expression of said protein.

**2.** Process according to claim 1, wherein the Morbillivirus is selected from among the virus of measles, the virus of Carre's disease, the virus of measles, the virus of cattle plague and the virus of small ruminants pest.

**3.** Process according to one of claims 1 to 2, comprising the virus of the vaccine.

**4.** Process according to claim 3, wherein the DNA sequence is under the control of a promoter of the vaccine.

**5.** Process according to claim 4, wherein the promoter of the vaccine is the 7.5 Kd promoter of the vaccine.

**6.** Process according to claim 5, wherein the one or more DNA sequences are cloned in the TK gene of the vaccine.

**7.** Process according to claim 5, wherein the one or more DNA sequences are cloned in the hr gene of the vaccine.

18

8. Process according to claim 5, comprising at least two DNA sequences coding for all or part of a structural protein of a Morbillivirus, said sequences being integrated in two regions of the genome selected from among the TK and hr genes.

9. Process for the preparation of all or part of a structural protein of a Morbillivirus, said process comprising cultivating mammalian cells infected with the poxvirus obtained by a process according to any one of claims 1 to 8 and recovering said structural protein.

10. Process for preparing a vaccine characterized in that it comprises a poxvirus obtained according to a process according to any one of claims 1 to 8 and/or proteins obtained by a process according to claim 9 alone or in combination, and/or a recombinant poxvirus, characterized in that it comprises, in one or several non essential parts of its genome, at least one DNA sequence coding for all or part of at least one HA hemagglutinating protein of a Morbillivirus as well as the elements ensuring the expression of the said protein.

11. Process according to claim 10, wherein the poxvirus is living.

12. Process according to claim 10, wherein the poxvirus is inactiated.

13. Process according to claim 10 to 12, characterized in that the poxvirus is the virus of the vaccine.

14. Process according to claim 13, characterized in that the DNA sequence is under the control of a promoter of the vaccine.

15. Process according to claim 14, characterized in that the promoter of the vaccine is the 7.5 Kd promoter.

16. Process according to claim 15, characterized in that the one or more DNA sequences are cloned in the TK gene of the vaccine.

17. Process according to claim 15, characterized in that the one or more DNA sequences are cloned in the hr gene of the vaccine.

18. Process according to claim 15, characterized in that it comprises at least two DNA sequences coding for all or part of a structural protein of a Morbillivirus, said sequences being integrated in two regions of the genome selected among the TK and hr genes.

**Patentansprüche**

**Patentansprüche für folgende Vertragsstaaten : AT, BE, CH, LI, DE, FR, GB, IT, LU, NL, SE**

1. Rekombinantes Poxvirus (Pockenvirus), dadurch gekennzeichnet, daß es in einem oder mehreren nicht-essentiellen Teilen seines Genoms mindestens eine DNA-Sequenz, die für einen Teil oder die Gesamtheit mindestens eines F- und/oder NP-Proteins eines Morbillivirus codiert, sowie seine Elemente, welche die Expression des genannten Proteins gewährleisten, enthält.

2. Poxvirus nach Anspruch 1, dadurch gekennzeichnet, daß das Morbillivirus ausgewählt wird aus dem Masern-Virus, dem Carré-Krankheits-Virus, dem Rinderpest-Virus und dem Kleinwiederkäuerpest-Virus ausgewählt wird.

3. Poxvirus nach Anspruch 1 oder 2, dadurch gekennzeichnet, daß es sich dabei um das Vaccine-Virus (Kuhpocken-Virus) handelt.

4. Poxvirus nach Anspruch 3, dadurch gekennzeichnet, daß die DNA-Sequenz unter der Kontrolle eines Promotors des Vaccine-Virus steht.

5. Poxvirus nach Anspruch 4, dadurch gekennzeichnet, daß es sich bei dem Promotor für das Vaccine-Virus um den Promotor 7,5 Kd des Vaccine-Virus handelt.

**6.** Poxvirus nach Anspruch 5, dadurch gekennzeichnet, daß die DNA-Sequenz(en) in das TK-Gen des Vaccine-Virus kloniert ist (sind).

**7.** Poxvirus nach Anspruch 5, dadurch gekennzeichnet, daß die DNA-Sequenz(en) in das hr-Gen des Vaccine-Virus kloniert ist (sind).

**8.** Poxvirus nach Anspruch 5, dadurch gekennzeichnet, daß es mindestens zwei DNA-Sequenzen aufweist, die für einen Teil oder die Gesamtheit eines Struktur-Proteins eines Morbillivirus codieren, wobei diese Sequenzen in zwei Bereiche des Genoms integriert sind, die ausgewählt werden aus den TK- und hr-Genen.

**9.** Rekombinante DNA, die dem Poxvirus (Pockenvirus) nach einem der Ansprüche 1 bis 8 entspricht.

**10.** Säugetier-Zelle, die mit dem Poxvirus (Pockenvirus) nach einem der Ansprüche 1 bis 8 infiziert ist.

**11.** Verfahren zur Herstellung der Gesamtheit oder eines Teils eines Strukturproteins eines Morbillivirus, dadurch gekennzeichnet, daß man Zellen nach Anspruch 10 kultiviert (züchtet) und das genannte Strukturprotein daraus gewinnt.

**12.** Morbillivirus-Strukturprotein, wie es nach dem Verfahren nach Anspruch 9 erhältlich ist.

**13.** Proteine nach Anspruch 12 und hämagglutinierende Proteins HA eines Morbillivirus jeweils allein oder in Form einer Kombination als immunogenes Agens.

**14.** Antiserum, das die gegen das Poxvirus (Pockenvirus) nach einem der Ansprüche 1 bis 8 oder gegen die Proteine nach Anspruch 12 gerichteten Antikörper enthält.

**15.** Impfstoff, dadurch gekennzeichnet, daß er ein Poxvirus (Pockenvirus) nach einem der Ansprüche 1 bis 8 und/oder immunogene Agentien nach Anspruch 13 jeweils allein oder in Form einer Kombination und/oder ein rekombinantes Poxvirus (Pockenvirus) enthält, das dadurch gekennzeichnet ist, daß es in einem oder mehreren nicht-essentiellen Teilen seines Genoms mindestens eine DNA-Sequenz, die für die Gesamtheit oder einen Teil mindestens eines hämagglutinierenden Proteins HA eines Morbillivirus codiert, sowie seine Elemente, welche die Expression des genannten Proteins gewährleisten, enthält.

**16.** Impfstoff nach Anspruch 15, dadurch gekennzeichnet, daß das Poxvirus (Pockenvirus) lebend ist.

**17.** Impfstoff nach Anspruch 15, dadurch gekennzeichnet, daß das Poxvirus (Pockenvirus) inaktiviert ist.

**18.** Impfstoff nach einem der Ansprüche 15 bis 17, dadurch gekennzeichnet, daß das Poxvirus (Pockenvirus) das Vaccine-Virus (Kohpocken-Virus) ist.

**19.** Poxvirus nach Anspruch 18, dadurch gekennzeichnet, daß die DNA-Sequenz unter der Kontrolle eines Promotors des Vaccine-Virus steht.

**20.** Impfstoff nach Anspruch 19, dadurch gekennzeichnet, daß es sich bei dem Promotor des Vaccine-Virus um den Promotor 7,5 Kd des Vaccine-Virus handelt.

**21.** Impfstoff nach Anspruch 20, dadurch gekennzeichnet, daß die DNA-Sequenz(en) in das TK-Gen des Vaccine-Virus kloniert ist (sind).

**22.** Impfstoff nach Anspruch 20, dadurch gekennzeichnet, daß die DNA-Sequenz(en) in das hr-Gen des Vaccine-Virus kloniert ist (sind).

**23.** Impfstoff nach Anspruch 20, dadurch gekennzeichnet, daß er mindestens zwei DNA-Sequenzen enthält, die für die Gesamtheit oder einen Teil eines Strukturproteins eines Morbillivirus codieren, wobei diese Sequenzen in zwei Bereiche des Genoms integriert sind, die ausgewählt werden aus den TK- und hr-Genen.

EP 0 305 229 B1

**Patentansprüche für folgende Vertragsstaaten : ES, GR**

1. Verfahren zur Herstellung eines rekombinanten Poxvirus (Pockenvirus), dadurch gekennzeichnet, daß man in einen oder mehrere nicht-essentielle Teile seines Genoms mindestens eine DNA-Sequenz, die für die Gesamtheit oder einen Teil mindestens eines F- und/oder NP-Proteins eines Morbillivirus codiert, sowie seine Elemente, welche die Expression des genannten Proteins gewährleistet, einführt.

2. Verfahren nach Anspruch 1, dadurch gekennzeichnet, daß das Morbillivirus ausgewählt wird aus dem Masern-Virus, dem Carré-Krankheits-Virus, dem Rinderpest-Virus und dem Kleinwiederkäuerpest-Virus.

3. Verfahren nach Anspruch 1 oder 2, dadurch gekennzeichnet, daß es sich bei dem Virus um das VaccineVirus (Kohpocken-Virus) handelt.

4. Verfahren nach Anspruch 3, dadurch gekennzeichnet, daß die DNA-Sequenz unter der Kontrolle eines Promotors des Vaccine-Virus steht.

5. Verfahren nach Anspruch 4, dadurch gekennzeichnet, daß es sich bei dem Promotor des Vaccine-Virus um den Promotor 7,5 Kd des Vaccine-Virus handelt.

6. Verfahren nach Anspruch 5, dadurch gekennzeichnet, daß die DNA-Sequenz(en) in das TK-Gen des Vaccine-Virus kloniert ist (sind).

7. Verfahren nach Anspruch 5, dadurch gekennzeichnet, daß die DNA-Sequenz(en) in das hr-Gen des Vaccine-Virus kloniert ist (sind).

8. Verfahren nach Anspruch 5, dadurch gekennzeichnet, daß das Poxvirus mindestens zwei DNA-Sequenzen enthält, die für die Gesamtheit oder einen Teil eines Strukturproteins eines Morbillivirus codieren, wobei diese Sequenzen in zwei Bereiche des Genoms integriert sind, die ausgewählt werden aus den TK- und hr-Genen.

9. Verfahren zur Herstellung der Gesamtheit oder eines Teils eines Strukturproteins eines Morbillivirus, dadurch gekennzeichnet, daß man Säugetierzellen, die mit einem Poxvirus (Pockenvirus) infiziert sind, wie es nach einem Verfahren nach einem der Ansprüche 1 bis 8 erhalten wird, kultiviert (züchtet), und daß man das genannte Strukturprotein daraus gewinnt.

10. Verfahren zur Herstellung eines Impfstoffes, dadurch gekennzeichnet, daß er ein Poxvirus (Pockenvirus), wie es bei einem Verfahren nach einem der Ansprüche 1 bis 8 erhalten wird, und/oder Proteine, die nach einem Verfahren nach Anspruch 9 erhalten werden, allein oder in Form einer Kombination und/oder ein rekombinantes Poxvirus (Pockenvirus) enthält, das dadurch gekennzeichnet ist, daß es in einem oder mehreren nicht-essentiellen Teilen seines Genoms mindestens eine DNA-Sequenz, die für die Gesamtheit oder einen Teil mindestens eines hämaglutinierenden Proteins HA eines Morbillivirus codiert, sowie seine Elemente, welche die Expression des genannten Proteins gewährleisten, enthält.

11. Verfahren nach Anspruch 10, dadurch gekennzeichnet, daß das Poxvirus (Pockenvirus) lebend ist.

12. Verfahren nach Anspruch 10, dadurch gekennzeichnet, daß das Poxvirus (Pockenvirus) inaktiviert ist.

13. Verfahren nach einem der Ansprüche 10 bis 12, dadurch gekennzeichnet, daß das Poxvirus das Vaccine-Virus (Kohpoacken-Virus) ist.

14. Verfahren nach Anspruch 13, dadurch gekennzeichnet, daß die DNA-Sequenz unter der Kontrolle eines Promotors des Vaccine-Virus steht.

15. Verfahren nach Anspruch 14, dadurch gekennzeichnet, daß der Promotor des Vaccine-Virus der Promotor 7,5 Kd des Vaccine-Virus ist.

16. Verfahren nach Anspruch 15, dadurch gekennzeichnet, daß die DNA-Sequenz(en) in das TK-Gen des Vaccine-Virus kloniert ist (sind).

21

**17.** Verfahren nach Anspruch 15, dadurch gekennzeichnet, daß die DNA-Sequenz(en) in das hr-Gen des Vaccine-Virus kloniert ist (sind).

**18.** Verfahren nach Anspruch 15, dadurch gekennzeichnet, daß das Poxvirus mindestens zwei DNA-Sequenzen enthält, die für die Gesamtheit oder einen Teil eines Strukturproteins eines Morbillivirus codieren, wobei diese Sequenzen in zwei Bereiche des Genoms integriert sind, die ausgewählt werden aus den TK- und hr-Genen.

FIG_1

FIG_2

## FIG_3

A

B

FIG_4

# FIG.5

FIG. 6

FIG_7

FIG_8

EP 0 305 229 B1

FIG_9

FIG_10

*VV*  *VV 2139*

*T*  *1*  *2*  *3*

NP

## FIG.11